# EUROPEAN PATENT APPLICATION

(11) **EP 3 184 102 A1**
(43) Date of publication of application: **28.06.2017**
(21) Application number: 15202426.1
(22) Date of filing: 23.12.2015
(51) Int. Cl.: A61K 9/16, A61K 9/50, A61K 31/4465

(54) **PHARMACEUTICAL COMPOSITION OF VORTIOXETINE HYDROBROMIDE COMPRISING AN INERT CORE FORMED OF AN ACIDIC REACTING COMPOUND**

(71) Applicant: Hexal AG, 83607 Holzkirchen (DE)
(72) Inventor: Oppelt, Renate., 83607 Holzkirchen (DE); Krekeler, Andreas., 83607 Holzkirchen (DE); Wawra, Christian., 83607 Holzkirchen (DE)
(74) Representative: Kluschanzoff, Harald

(57) **Abstract**

The present invention relates to a solid oral pharmaceutical composition comprising an inert core comprising, preferably consisting of, at least one acidic reacting compound, and a coating comprising vortioxetine hydrobromide and at least one pharmaceutically acceptable polymer and a method for enhancing the solubility and bioavailability of vortioxetine hydrobromide.

## Description

### FIELD OF THE INVENTION

The present invention relates to a pharmaceutical composition comprising vortioxetine hydrobromide, a process for the preparation thereof, use thereof and a method for enhancing the bioavailability of vortioxetine hydrobromide.

### BACKGROUND OF THE INVENTION

Vortioxetine (also known under the name Lu-AA21004 and the tradename Brintellix®) has the chemical name 1-{2-[(2,4-dimethylphenyl)sulfanyl]phenyl}piperazine, the structure of which is

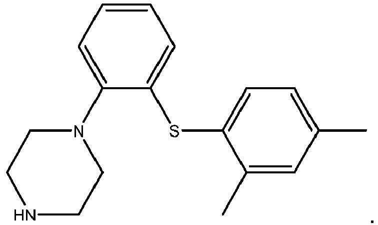

Recently, the FDA approved vortioxetine for the treatment of adults with major depressive disorder. The finished product is presented as immediate-release film-coated tablet containing 5 to 20mg of Vortioxetine (as crystalline hydrobromide salt) as the active substance. Vortioxetine exhibits polymorphism and appears in several polymorphs. A method of producing crystalline vortioxetine and several acid addition salts thereof is disclosed in WO 2007/144005 A1.

Nausea is reported to be the most common adverse reaction and its frequency was dose-related (cf. Prescribing Information Brintellix®). According to WO 2011/023194, the amount of adverse events can be lowered, if vortioxetine is not released in the stomach but in the intestine (enteric coated tablets or enteric coated multiparticulate compositions are suggested).

However, vortioxetine hydrobromide is only slightly soluble in water and aqueous solutions, and the inventors found that the solubility of vortioxetine hydrobromide decreases at higher pH (such as in the intestine fluid). Hence, there is the need to find a pharmaceutical composition which is connected with a lowered occurrence of side effects, but at the same time has an improved bioavailability.

It has been unexpectedly found that the solubility and bioavailability of vortioxetine hydrobromide is improved (in particular, at higher pH) if the vortioxetine hydrobromide is provided in a pharmaceutical composition which comprises an inert core comprising, and preferably consisting of, at least one acidic reacting compound, and furthermore a coating comprising vortioxetine hydrobromide and at least one pharmaceutically acceptable polymer. This finding is particularly surprising since the enteric coatings of the prior art compositions usually are already compounds containing free carboxylic acid groups on the polymer backbone and there was no indication that providing further acidic compounds could be advantageous.

Based on these findings, the inventors developed pharmaceutical compositions which provide an excellent balance between reduced adverse events and solubility and bioavailability of vortioxetine hydrobromide.

The present invention thus provides an oral solid pharmaceutical composition comprising an inert core comprising, and preferably consisting of, at least one acidic reacting compound, and a coating comprising vortioxetine hydrobromide and at least one pharmaceutically acceptable polymer. Preferably, said pharmaceutical composition is gastro-resistant, i.e. is intended to resist the gastric fluid and to release vortioxetine hydrobromide in the intestinal fluid.

The present invention also provides a process for the preparation of the solid oral pharmaceutical composition as described herein, wherein said process comprises the steps of preparing a suspension or solution of vortioxetine hydrobromide and the at least one pharmaceutically acceptable polymer and optionally one or more pharmaceutical excipients, and coating a pellet or crystal comprising, preferably consisting of, at least one acidic reacting compound with the suspension or solution.

The present invention furthermore provides an solid oral pharmaceutical composition as described herein for use in the treatment of a disease selected from affective disorders, depression, major depressive disorder, postnatal depression, depression associated with bipolar disorder, Alzheimer's disease, psychosis, cancer, age or Parkinson's disease, anxiety, general anxiety disorder, social anxiety disorder, obsessive compulsive disorder, panic disorder, panic attacks, phobia, social phobia, agoraphobia, stress urinary incontinence, emesis, irritable bowel syndrome, eating disorders, chronic pain, partial responders, treatment resistant depression, Alzheimer's disease, cognitive impairment, attention deficit hyperactivity disorder, melancholia, posttraumatic stress disorder, hot flushes, sleep apnea, alcohol, nicotine or carbohydrate craving, substance abuse and alcohol and drug abuse.

The present invention also provides a method for enhancing the bioavailability of vortioxetine hydrobromide, characterized in that the vortioxetine hydrobromide is provided in a solid oral pharmaceutical composition as described herein.

### DETAILED DESCRIPTION OF THE INVENTION

The present invention provides an oral solid pharmaceutical composition comprising an inert core comprising, and preferably consisting of, at least one acidic reacting compound, and a coating comprising vortioxetine hydrobromide and at least one pharmaceutically acceptable polymer.

The term "Vortioxetine hydrobromide" means the pharmaceutically acceptable acid addition salt of vortioxetine with hydrochlorid acid. Pharmaceutically acceptable salts are intended to indicate acid addition salts of acids that are non-toxic. Said salts include salts made from organic acids, such as maleic, fumaric, benzoic, ascorbic, succinic, oxalic, bis-methylenesalicylic, methanesulfonic, ethanedisulfonic, acetic, propionic, tartaric, salicylic, citric, gluconic, lactic, malic, mandelic, cinnamic, citraconic, aspartic, stearic, palmitic, itaconic, glycolic, p-aminobenzoic, glutamic, benzenesulfonic, theophylline acetic acids, as well as the 8-halotheophyllines, for example 8-bromotheophylline. Said salts may also be made from inorganic acids, such as hydrochloric, hydrobromic, sulfuric, sulfamic, phosphoric and nitric acids. Although distinct mention is made of the hydrobromide acid salt, the composition of the present invention can comprise any pharmaceutically acceptable salt of vortioxetine instead of salts made from hydrobromic acid. Particular mention is made of salts made from lactic acid. Particular mention is also made of salts made from acetic acid.

The vortioxetine hydrobromide used in the pharmaceutical composition of the present invention can be crystalline or amorphous.

WO 2007/144005 discloses polymorph forms alpha and beta, and WO 2014/044721 discloses a further crystalline form of vortioxetine hydrobromide having an XRPD pattern with characteristic peaks (expressed in 2θ ± 0,2° 2θ (CuKα radiation)) at 5.5°, 14.8°, 16.7° and 20.0°. The non-prepublished copending patent application PCT/EP2015/074607 describes amorphous forms of vortioxetine hydrobromide and methods for the production. WO 2007/144005 describes a method for the production of vortioxetine hydrobromide.

Generally any crystalline form of vortioxetine hydrobromide, such as forms alpha, beta and the above-mentioned form disclosed in WO 2014/044721, and the amorphous form of vortioxetine hydrobromide can be used to prepare the pharmaceutical composition of the invention. In the pharmaceutical composition of the present invention, the vortioxetine hydrobromide is preferably present in the amorphous form.

The abbreviation (w/w) as used herein refers to weight/weight percentages.

As used herein, the term "amorphous" means a solid body devoid of long-range crystalline order. Such a lack of crystalline order can be detected and monitored, e.g., by X-ray diffraction (XRD), FT-Raman spectroscopy, and differential scanning calorimetry (DSC).

As used herein, the phrase "amorphous vortioxetine hydrobromide" means that the vortioxetine hydrobromide is in the amorphous state, e.g., that there is a minimum of 95% of vortioxetine hydrobromide in the amorphous state, preferably 98% and more preferably 99% or more, or even 100%.

Vortioxetine hydrobromide in the amorphous form has an improved solubility compared to the crystalline form. Moreover, amorphous vortioxetine hydrobromide which is embedded in the pharmaceutical acceptable polymer in the coating is stable and does not show the tendency of polymorphic conversion. Therefore, it is advantageous to provide the pharmaceutical composition of the present invention with the amorphous form of vortioxetine hydrobromide.

Preferably, the vortioxetine hydrobromide is present in an amount of 1-20% (w/w), more preferably 1-15% (w/w), and particularly preferably 1-10% (w/w) in the pharmaceutical composition of the present invention, each based on the total weight of the composition.

Preferably, the solid oral pharmaceutical composition of the present invention is gastro-resistant. The term "gastro-resistant" means a delayed-release solid oral pharmaceutical composition that is intended to resist the gastric fluid and to release their active substance(s) in the intestinal fluid. Tablets covered with a gastro-resistant coating conform to the definition of coated tablets.

Testing whether a pharmaceutical composition is gastro-resistant can be done e.g. using the USP Dissolution Apparatus 2 (Paddle). With respect to details of the process it is referred to the dissolution tests described in the US Pharmacopeial Convention <711> and/or the European Pharmacopeia (2.9.3.). According to Method B, the pharmaceutical composition is placed in 1000 mL 0.1 N hydrochloric acid, stirred at a temperature of 37±0.5 °C for 2 hours. An aliquot of the solution is taken. Next, the pharmaceutical composition is placed in 1000 mL of 0.20 M phosphate buffer, stirred at a temperature of 37±0.5 °C and a pH of 6.8±0.05 for 45 minutes (or for the specified time). An aliquot of the solution is taken. Then, the amount of the active ingredient in both aliquots is measured in a suitable assay.

The term "release rate" or "release profile" refers to the (percentage) amount released per time unit from the dosage form comprising the pharmaceutical composition. For the purpose of the invention of this invention, the in-vivo release profile will be assumed as corresponding to the in-vitro dissolution profile as explained below and will be measured as explained below and in the examples by means of the in-vitro dissolution rate.

As used herein, the terms "gastro-resistant" or "substantially insoluble in the stomach of a human patient" mean that not more than 40% (w/w), preferably not more than 30% (w/w), more preferably not more than 20% (w/w), and most preferably not more than 10% (w/w) of the vortioxetine hydrobromide contained in the analyzed pharmaceutical composition, based on the total weight amount of vortioxetine hydrobromide in the pharmaceutical composition, can be found in the first (acidic) solution tested as described above (i.e. stirred in 0.1 N hydrochloric acid at a temperature of 37±0.5 °C for 2 hours). The pharmaceutical composition is "soluble in the intestine", if at least 50% (w/w), preferably at least 60% (w/w) of the vortioxetine hydrobromide contained in the analyzed pharmaceutical composition can be found in the second (non-acidic) solution tested as described above (i.e. stirred in phosphate buffer at a temperature of 37±0.5 °C and a pH of 6.8±0.05 for 1 hour). It is preferred that all of the vortioxetine hydrobromide that is not dissolved in the HCl environment is dissolved in the environment with the pH of 6.8. Preferably, the pharmaceutical composition according to the invention fulfills the criteria for the dissolution test of solid dosage forms with delayed release according to the European Pharmacopeia.

The pharmaceutical composition of the present invention comprises a coating, wherein the coating comprises at least one pharmaceutically acceptable polymer, and vortioxetine hydrobromide.

The term "polymer" as used herein encompasses also copolymers formed from two or more monomers. The coating can comprise more than one pharmaceutically acceptable polymer, however, it is preferred that the coating is formed from one polymer.

The pharmaceutical composition of the present invention is preferably gastro-resistant, i.e. substantially insoluble in the stomach (measured with the in vitro method as described above). Preferably, substantially no (or only minor amounts of) vortioxetine hydrobromide is released into the stomach, where vortioxetine hydrobromide leads to most of the described adverse events, such as e.g. nausea. In view of the aforesaid, the polymer forming the coating is preferably an enteric polymer, which preferably comprises free carboxylic acid groups.

The (enteric) polymer can be any of the known (enteric) coating polymers which are used in the pharmaceutical field and are known to a skilled person. Herein, the (enteric) polymer is used for forming a coating on an inert core comprising, and preferably consisting of, at least one acidic reacting compound. However, this does not exclude the possibility that between the inert core and the coating of the pharmaceutical composition a further inert layer is present.

Examples of the (enteric) polymer include, but are not limited to cellulose based polymers and methacrylic acid copolymers.

Examples of cellulose based polymers include, but are not limited to cellulose acetate succinate, hydroxy propyl methyl cellulose phthalate (e.g. HPMCP®, Shin-Etsu Chemical Co., Ltd., Tokyo, Japan), hydroxy propyl methyl cellulose acetate succinate (HPMCAS, e.g. AQOAT®, Shin-Etsu Chemical Co., Ltd., Tokyo, Japan) and cellulose acetate phthalate. Particularly preferred is hydroxy propyl methyl cellulose acetate succinate (HPMCAS).

Examples of methacrylic acid copolymers include, but are not limited to Poly(methacrylic acid-co-ethyl acrylate), Poly(methacrylic acid-co-methyl methacrylate), Poly(methacrylic acid-co-methyl methacrylate) and Poly(methyl acrylate-co-methyl methacrylate-co-methacrylic acid), which are sold e.g. as Eudragit® L30 D-55, Eudragit® L100-55, Eudragit® L100, Eudragit® L12,5, Eudragit® S100, Eudragit® S12,5 and Eudragit® FS 30D (Evonik Degussa Corporation, Parsipanny, NJ). Further examples of such polymers include Kollicoat MAE 30 DP and Kollicoat MAE 100P (BASF, Germany).

In a particularly preferred embodiment, the at least one pharmaceutically acceptable enteric polymer comprises HPMCAS and/or poly(methacrylic acid-co-methyl methacrylate) with a 1:1 ratio of methacrylic acid to methyl methacrylate.

The coating of the pharmaceutical composition of the present invention preferably consists of a solid solution or solid dispersion of the vortioxetine hydrobromide embedded in the pharmaceutically acceptable (enteric) polymer. In this embodiment, the vortioxetine hydrobromide is dissolved or dispersed in the (enteric) polymer which forms the continuous phase of the solid solution or solid dispersion. Optionally, the solid solution or solid dispersion comprises one or more pharmaceutically acceptable excipients.

In one embodiment of the invention, the vortioxetine hydrobromide is present in the coating in an amount of 1-50 % (w/w) based on the total weight of the coating. In a preferred embodiment, the vortioxetine hydrobromide is present in the coating an amount of 2-45 % (w/w) based on the total weight of the coating. In another embodiment, the vortioxetine hydrobromide is present in the coating in an amount of 2-40 % (w/w), preferably 3-35 % (w/w), and particularly preferably 5-30 % (w/w) based on the total weight of the coating. In the overall pharmaceutical composition, the vortioxetine hydrobromide is preferably present in an amount of 1-25 % (w/w), 1-20 % (w/w) or 1-15 % (w/w) based on the total weight of the pharmaceutical composition.

In a further embodiment of the invention, the pharmaceutically acceptable (enteric) polymer is present in the coating in an amount of 20-70 % (w/w) based on the total weight of the coating. In a preferred embodiment, the pharmaceutically acceptable (enteric) polymer is present in the coating in an amount of 20-65 % (w/w) based on the total weight of the coating. In another embodiment, the pharmaceutically acceptable (enteric) polymer is present in the coating in an amount of 25-60 % (w/w), preferably 25-55 % (w/w), and particularly preferably 30-50 % (w/w) based on the total weight of the coating.

In the overall pharmaceutical composition, the (enteric) polymer is preferably present in an amount of 1-40 % (w/w) based on the total weight of the pharmaceutical composition. In a preferred embodiment, the pharmaceutically acceptable (enteric) polymer is present in the composition in an amount of 2-35 % (w/w) based on the total weight of the pharmaceutical composition. In another embodiment, the pharmaceutically acceptable (enteric) polymer is present in the composition in an amount of 2-30 % (w/w), preferably 2-25 % (w/w), and particularly preferably 2-20 % (w/w) based on the total weight of the composition.

The weight ratio of vortioxetine hydrobromide to the at least one pharmaceutically acceptable (enteric) polymer in the coating is preferably 1 : 1-5, more preferably 1 : 1-3, and particularly preferably 1 : 2.

The coating comprising the vortioxetine hydrobromide and the at least one pharmaceutically acceptable polymer is preferably present in the pharmaceutical composition of the invention in an amount of 1-50 % (w/w) based on the total weight of the pharmaceutical composition. In a further preferred embodiment, the coating is present in an amount of 2-45 % (w/w) based on the total weight of the pharmaceutical composition. In another preferred embodiment, the coating is present in an amount of 3-40 % (w/w), preferably 4-35 % (w/w), and most preferably 5-30 % (w/w) based on the total weight of the pharmaceutical composition.

The coating of the pharmaceutical composition of the present invention can consist exclusively of vortioxetine hydrobromide and the at least one pharmaceutically acceptable (enteric) polymer. However, in a further preferred embodiment, the coating contains vortioxetine hydrobromide, the at least one pharmaceutically acceptable (enteric) polymer and one or more further acceptable excipients.

Said one or more further pharmaceutically acceptable excipients are usual pharmaceutically acceptable excipients, and examples of such excipients include, but are not limited to antioxidants, binders, bulking agents, disintegrants, fillers, glidants, lubricants, surfactants and plasticizers. The pharmaceutically acceptable excipients are preferably contained in the coating in an amount of 0.01-70% (w/w), more preferably in an amount of 0.1-65% (w/w), even more preferably in an amount of 1-60% (w/w), and particularly preferably in an amount of 5-55% (w/w) based on the total weight of the coating.

Examples of antioxidants include water soluble antioxidants such as ascorbic acid, sodium sulfite, metabisulfite, sodium miosulfite, sodium formaldehyde, sulfoxylate, isoascorbic acid, isoascorbic acid, cysteine hydrochloride, 1,4-diazobicyclo-(2,2,2)-octane, and mixtures thereof. Examples of oil-soluble antioxidants include ascorbyl palmitate, butylated hydroxyanisole, butylated hydroxytoluene, potassium propyl gallate, octyl gallate, dodecyl gallate, phenyl-α-napthyl-amine, and tocopherols such as α-tocopherol.

Examples of binders include, but are not limited to, starches, celluloses and derivatives thereof, sucrose, dextrose, corn syrup, polysaccharides, and gelatin. Examples of celluloses and derivatives thereof include for example, microcrystalline cellulose, e.g., AVICEL PH from FMC (Philadelphia, PA).

Examples of bulking agents include, without limitation, PEGs, mannitol, trehalose, lactose, sucrose, sucrose, glycine, cyclodextrins, dextran and derivatives and mixtures thereof. Especially preferred is mannitol, e.g. PEARLITOL® 50C from Roquette Pharma (Lestrem, France).

Examples of disintegrants include, but are not limited to starches, e.g. sodium carboxymethyl starch or sodium starch glycolate; clays; alginates; gums; cross-linked polymers, e.g., cross-linked polyvinyl pyrrolidone or crospovidone, e.g., POLYPLASDONE XL from International Specialty Products (Wayne, NJ); cross-linked sodium carboxymethylcellulose or croscarmellose sodium, e.g., AC-DI-SOL from FMC; and cross-linked calcium carboxymethylcellulose; soy polysaccharides; and guar gum. Especially preferred is sodium starch glycolate, e.g. PRIMOJEL® from DFE-Pharma (Goch, Germany).

Examples of pharmaceutically fillers include, but are not limited to confectioner's sugar, compressible sugar, dextrates, dextrin, dextrose, lactose, mannitol, microcrystalline cellulose, powdered cellulose, sorbitol, sucrose and talc.

Examples of glidants and lubricants include, but are not limited to, colloidal silica, magnesium trisilicate, starches, talc, tribasic calcium phosphate, magnesium stearate, aluminum stearate, calcium stearate, magnesium carbonate, magnesium oxide and polyethylene glycol. Especially preferred is talc.

Surfactants include, but are not limited to, fatty acid and alkyl sulfonates; benzethonium chloride, e.g., HYAMINE 1622 from Lonza, Inc. (Fairlawn, NJ); polyoxyethylene sorbitan fatty acid esters, e.g., the TWEEN Series from Uniqema (Wilmington, DE); and natural surfactants, such as sodium taurocholic acid, 1-palmitoyl-2-Sn-glycero-3-phosphocholine, lecithin and other phospholipids.

An example for a plasticizer is triethyl citrate. In a preferred embodiment, the coating of the pharmaceutical composition of the present invention further contains triethyl citrate and talc.

Besides the coating described above, the pharmaceutical composition of the present invention comprises an inert core. Said inert core comprises, and preferably consists of at least one acidic reacting compound. The acidic reacting compound is a pharmaceutically acceptable acidic reacting compound. The term "inert" as used herein means that the core does not chemically react with the vortioxetine hydrobromide or the at least one pharmaceutically acceptable (enteric) polymer contained in the coating of the pharmaceutical composition during the preparation and storage of the pharmaceutical composition.

The term "acidic reacting compound" as used herein means that if the compound is dissolved or dispersed in a suitable solvent, such as water, the solution provides a pH value of 6.0 or lower, preferably 5.0 or lower measured at room temperature. For this test, 1 g of the acidic reacting compound is dissolved or dispersed in 100 mL water and the pH is measured after 5 minutes.

The acidic reacting compound can be any of the known acidic reacting compounds, such as organic and inorganic acids, which are used in the pharmaceutical field and known to a skilled person. In a preferred embodiment the acidic reacting compound is any of the known organic acids which are used in the pharmaceutical field and known to a skilled person.

Examples of the at least one acidic reacting compound include, but are not limited to tartaric acid, fumaric acid, succinic acid, citric acid, malic acid, glutamic acid, aspartic acid or one of the hydrates thereof. Especially preferred is tartaric acid as the inert core.

In one embodiment, the inert core is provided as a pellet formed of the at least one acid reacting compound. In such a pharmaceutical composition, the pellet core is coated with the coating comprising vortioxetine hydrobromide and at least one pharmaceutically acceptable (enteric) polymer as described above. Optionally, the pellet core is separated from the vortioxetine hydrobromide containing coating by an inert layer. Pellet cores from acidic reacting compounds, e.g. from tartaric acid, are known in the art and disclosed e.g. in WO 03/074056.

Instead of a pellet, the core can also consist of a crystal of one acidic reacting compound such as a crystal of tartaric acid or citric acid.

It is preferred that the at least one acidic reacting compound is present in the pharmaceutical composition of the invention in an amount of 40-95 % (w/w), based on the total weight of the composition. In a more preferred embodiment, the coating is present in the pharmaceutical composition of the invention an amount of 45-90 % (w/w) based on the total weight of the pharmaceutical composition. In further preferred embodiment, the coating is present in an amount of 50-85 % (w/w), preferably 55-80 % (w/w), and most preferably 60-80 % (w/w) based on the total weight of the pharmaceutical composition.

The weight ratio of vortioxetine hydrobromide to the at least one acidic reacting compound in the pharmaceutical composition of the present invention is preferably 1 : 2-25, more preferably 1 : 5-20, and particularly preferably 1 : 10-15.

The pharmaceutical composition of the present invention can consist exclusively of the inert core comprising, preferably consisting of, the at least one acidic reacting compound as described above, and the coating comprising vortioxetine hydrobromide and at least one pharmaceutically acceptable (enteric) polymer as described above.

On the other hand, the pharmaceutical composition of the present invention can further comprise a usual enteric coating. Such enteric coatings are known in the art and prevent that active ingredient is released in the stomach. The pharmaceutical compositions of the present invention comprise a coating of a pharmaceutically acceptable polymer, wherein the vortioxetine hydrobromide is embedded, as described above. However, if it is not possible to provide a sufficient "enteric effect" by this coating alone, which means that too much vortioxetine hydrobromide is released from the pharmaceutical composition into the stomach, an enteric coating can additionally be present surrounding the coating of the pharmaceutical composition as described above.

The process for the preparation of the pharmaceutical composition according to the invention comprises the steps of preparing a suspension or solution of vortioxetine hydrobromide and the at least one pharmaceutically acceptable enteric polymer and optionally one or more pharmaceutical excipients, and coating a pellet or crystal comprising, preferably consisting of, at least one acidic reacting compound with the suspension or solution.

In a preferred process for the preparation of the pharmaceutical composition according to the invention, the vortioxetine hydrobromide is dissolved in a solvent, e.g. ethanol. Optionally, pharmaceutically acceptable excipients are dissolved e.g. in water, followed by combining the vortioxetine hydrobromide solution and the pharmaceutically acceptable excipients solution. The pharmaceutically acceptable (enteric) polymer is added to the resulting solution, which is stirred until a solution or homogenous suspension is formed. Optionally, further pharmaceutically acceptable excipients are added to the solution or suspension. The solution or suspension is then sprayed on a pellet or a crystal of the at least one acidic reacting compound, e.g. in a fluid bed Wurster device, resulting in the pharmaceutical composition of the present invention. The resulting composition can optimally be further coated e.g. with a usual enteric coating by means of techniques common in the art and known to the skilled person.

The pharmaceutical composition as described above can be used in the treatment of a disease selected from affective disorders, depression, major depressive disorder, postnatal depression, depression associated with bipolar disorder, Alzheimer's disease, psychosis, cancer, age or Parkinson's disease, anxiety, general anxiety disorder, social anxiety disorder, obsessive compulsive disorder, panic disorder, panic attacks, phobia, social phobia, agoraphobia, stress urinary incontinence, emesis, irritable bowel syndrome, eating disorders, chronic pain, partial responders, treatment resistant depression, Alzheimer's disease, cognitive impairment, attention deficit hyperactivity disorder, melancholia, posttraumatic stress disorder, hot flushes, sleep apnea, alcohol, nicotine or carbohydrate craving, substance abuse and alcohol or drug abuse.

For the above-mentioned indications, the appropriate dosage will vary depending on, for example, the host, the mode of administration, the nature and severity of the condition, disease or disorder or the effect desired. Vortioxetine hydrobromide may be conveniently administered in a unit dose form comprising 1-50 mg of vortioxetine hydrobromide. The total daily dose is expected to be in the range of 5-20 mg of vortioxetine hydrobromide.

The present invention also comprises a method for enhancing the solubility of vortioxetine hydrobromide, characterized in that the vortioxetine hydrobromide is provided in a pharmaceutical composition as described above.

In the presently known pharmaceutically acceptable compositions which are enterically coated (in order to prevent side effects occurring if vortioxetine hydrobromide is released into the stomach), the enteric coating, which is usually a coating comprising polymers containing free carboxylic acid groups on the polymer backbone, is dissolved in the intestine. However, as mentioned above, inventors have found that vortioxetine hydrobromide is less soluble in the basic environment of the intestine than in the acidic environment if the stomach. In the conventional enterically coated formulations, the environment of the composition is yet rendered slightly acidic when the enteric coating is dissolved; however, it is no longer sufficiently acidic when the vortioxetine hydrobromide is released.

The pharmaceutical composition of the present invention, on the other hand, is soluble in the intestine because the core of the composition comprises, and preferably consists of at least one acidic reacting compound that provides an acidic pH-microclimate in the intestine. Hence, the vortioxetine hydrobromide is dissolved in an acidic pH-microclimate in the intestine, established by the acidic core.

Thus, the pharmaceutical composition according to the invention provides on the one hand a release profile that is favorable with regard to adverse events (less or even no amounts of vortioxetine hydrobromide is released in the stomach), and at the same time provides an acidic pH-microclimate at higher pH (such as in the intestine), improving the solubility of vortioxetine hydrobromide in the higher pH environment of the intestine, which leads to an enhanced solubility and bioavailability. Thus, enhancing the solubility and bioavailability also means that the solubility and bioavailability of vortioxetine hydrobromide is increased compared to a conventional enteric coated composition, which does not contain an inert core formed of an acidic reacting compound.

The pharmaceutical composition of the present invention is provided as solid oral dosage form. Solid oral dosage forms include, but are not limited to tablets, hard or soft capsules, caplets, lozenges, pills, mini-tablets, pellets, beads, granules (e.g. packaged in sachets).

Compositions for oral use are prepared according to any method known in the art for the manufacture of pharmaceutical compositions and such compositions can contain one or more agents selected from the group consisting of sweetening agents, flavoring agents, coloring agents and preserving agents in order to provide pharmaceutically elegant and palatable preparations.

The present invention will be explained in more detail with the following examples, which are not to be interpreted as limiting.

### Examples

### Example 1: Preparation of formulations with different core materials and polymers

88 g vortioxetine hydrobromide in the crystalline beta form (a method for the production is described in example 4c of WO 2007/144005) was dissolved in 1356 g ethanol at room temperature (20-30°C). 22 g trithylcitrate was dissolved in 594 g water at room temperature (20-30°C). The aqueous triethylcitrate solution was added to the vortioxetine hydrobromide solution and mixed until a clear solution was formed. 176 g HPMCAS-MG (Shin-Etsu Chemical Co. Ltd.) (Formulations A and B) and Eudragit L 100 (Formulation C) (Evonik Degussa Corporation) (Formulation C), respectively, was slowly added to this solution, which was stirred until the polymer dissolved. Afterwards, 114.40 g talc was suspended in the solution while stirring. The resulting suspension was sprayed on tartaric acid pellets (Formulation A) or saccharose pellets (Formulations B and C) in a fluid bed Wurster system by applying the following process parameters for Formulations A and B:

| | |
|---|---|
| Inlet air temperature: | 50-53 °C |
| Product temperature: | 39-44 °C |
| Spray rate: | 1-10 g/min, |

and the following parameters for Formulation C:

| | |
|---|---|
| Inlet air temperature: | 30-32 °C |
| Product temperature: | 25-28 °C |
| Spray rate: | 1-10 g/min. |

The composition of the resulting Formulations A, B and C is summarized in the following Table 1:

**Table 1**

| **Ingredient** | **Formulation A [mg] (inventive)** | **Formulation B [mg]** (**comparative**) | **Formulation C [mg]** (**comparative**) |
|---|---|---|---|
| Vortioxetine hydrobromide | 2.46 | 2.46 | 2.55 |
| HPMCAS-MG | 4.92 | 4.92 | - |
| Eudragit L 100 | - | - | 5.10 |
| Triethylcitrate | 0.61 | 0.61 | 0.51 |
| Talc | 3.20 | 3.20 | 2.55 |
| Tartaric acid pellets | 33.52 | - | - |
| Sugar Pellets | - | 33.52 | 34.73 |
| **Σ Formulation** | **44.71** | **44.71** | **45.44** |

Crystalline forms of vortioxetine hydrobromide within the pellets of Formulations A, B and C were checked via XRPD measurement. No signals relating to crystalline API were detected. All visible signals were attributed to tartaric acid, saccharose and talc.

### Example 2: Dissolution test

The pellets of Formulations A, B and C obtained in Example 1 were checked for the dissolution profile of vortioxetine hydrobromide, using the following parameters: 900 mL 0.05M KH₂PO₄, pH 6,8, paddle apparatus, 50 rpm, 37±0.5 °C. The results are summarized in the following Table 2:

**Table 2**

| **Time [min]** | **Formulation A [% (w/w) release vortioxetine hydrobromide**] | **Formulation B [% (w/w) release vortioxetine hydrobromide]** | **Formulation C [% (w/w) release vortioxetine hydrobromide**] |
|---|---|---|---|
| 0 | 0 | 0 | 0 |
| 5 | 15 | 6 | 0 |
| 10 | 38 | 10 | 0 |
| 15 | 44 | 13 | 0 |
| 30 | 57 | 23 | 1 |
| 45 | 70 | 32 | 2 |
| 60 | 79 | 41 | 2 |

Eudragit L 100 is soluble in neutral to weakly alkaline conditions (pH 6-7) and forms salts with alkalis, thus affording film coats which are resistant to gastric media but soluble in intestinal fluid. HPMCAS is a mixture of acetic acid and monosuccinic acid esters of hydroxypropylmethyl cellulose. HPMCAS is used as an enteric film coating because it is insoluble in acidic gastric fluid, but will swell and dissolve readily in the upper small intestine. It can also be used as a solid-dispersion carrier for poorly soluble compounds.

As may be taken from Table 2, after 60 min significantly more vortioxetine hydrobromide was released from Formulation A using an acidic tartaric acid core than from Formulations B and C using a non-acidic sugar core (79% (w/w) for Formulation A vs. 41% (w/w) for Formulation B and 2% (w/w) for Formulation C, each based on the amount of vortioxetine hydrobromide contained in the formulations).

Unexpectedly, the solubility of vortioxetine hydrobromide is greatly improved if amorphous vortioxetine hydrobromide is provided in a pharmaceutical composition which additionally comprises an acidic reacting compound as core. The results show that the acidic tartaric acid core (Formulation A) creates an acidic microenvironment wherein vortioxetine hydrobromide is readily dissolved.

### Example 3: Enteric coating

Formulations A and B of Example 2 are enteric coated with a film coating formulation according to the following composition.

| **Ingredient** | **Formulation A [mg] (inventive)** |
|---|---|
| HPMCAS-MG | 20.63 |
| Triethylcitrate | 2.06 |
| Talc | 10.31 |
| Σ **Coating** | **33.00** |

Trithylcitrate is dissolved in 66.3 mL water at room temperature (20-30°C). The aqueous triethylcitrate solution is added to 154.7 mL ethanol and mixed until a clear solution is formed. HPMCAS-MG (see Example 2) is slowly added to this solution, which is stirred until the polymer dissolved. Afterwards talc is suspended in the solution while stirring. The resulting suspension is sprayed on the coated pellets (Formulations A/B) in a fluid bed Wurster system by applying the process parameters as described in Example 2 herein above. The enteric coated formulation is substantially insoluble in an acidic medium (900 mL 0.1 N hydrochloric acid, stirred at 50 rpm at a temperature of 37±0.5 °C for 2 hours).

## Claims

1. A solid oral pharmaceutical composition comprising:
an inert core comprising, preferably consisting of, at least one acidic reacting compound,
and a coating comprising vortioxetine hydrobromide and at least one pharmaceutically acceptable polymer.

2. The pharmaceutical composition according to claim 1, wherein the vortioxetine hydrobromide is present in an amount of 1-20 % (w/w) based on the total weight of the composition.

3. The pharmaceutical composition according to claim 1 or 2, wherein the pharmaceutically acceptable polymer is an enteric polymer, preferably an enteric polymer comprising a cellulose based polymer and/or a methacrylic acid copolymer.

4. The pharmaceutical composition according to claim 3, wherein the pharmaceutically acceptable enteric polymer comprises HPMCAS, and/or poly(methacrylic acid-co-methyl methacrylate) with a 1:1 ratio of methacrylic acid to methyl methacrylate.

5. The pharmaceutical composition according to any one of the preceding claims, wherein the at least one pharmaceutically acceptable polymer is present in the coating in an amount of 20-70 % (w/w) based on the total weight of the coating, and the vortioxetine hydrobromide is present in the coating in an amount of 1-50 % (w/w) based on the total weight of the coating, and the coating optionally further contains one or more pharmaceutically acceptable excipients.

6. The pharmaceutical composition according to any one of the preceding claims, wherein the coating consists of a solid solution or solid dispersion of the vortioxetine hydrobromide in the pharmaceutically acceptable polymer and optionally one or more pharmaceutically acceptable excipients.

7. The pharmaceutical composition according to any one of the preceding claims, wherein the at least one acidic reacting compound is a compound selected from the group consisting of tartaric acid, fumaric acid, succinic acid, citric acid, malic acid, glutamic acid, aspartic acid or one of the hydrates thereof.

8. The pharmaceutical composition according any one of the preceding claims, wherein the at least one acidic reacting compound is present in an amount of 40-95 % (w/w) based on the total weight of the composition.

9. The pharmaceutical composition according to any one of the preceding claims, wherein the vortioxetine hydrobromide is amorphous.

10. The pharmaceutical composition according to any of the preceding claims, wherein the weight ratio of vortioxetine hydrobromide to the at least one pharmaceutically acceptable polymer in the coating is 1 : 1-5, preferably 1 : 1-3, and the weight ratio of vortioxetine hydrobromide to the at least one acidic reacting compound in the composition is 1 : 2-25, preferably 1 : 5-20.

11. The pharmaceutical composition according to any one of the preceding claims, wherein the pharmaceutical composition is gastro-resistant.

12. The pharmaceutical composition according to any of the preceding claims, further comprising at least one enteric coating layer surrounding the coating of the pharmaceutical composition.

13. A process for the preparation of the pharmaceutical composition according to any one of claims 1 to 12, comprising the steps of:
preparing a suspension or solution of vortioxetine hydrobromide and at least one pharmaceutically acceptable polymer and optionally one or more pharmaceutical excipients,
and coating a pellet or crystal comprising, preferably consisting of, at least one acidic reacting compound with the suspension or solution.

14. A pharmaceutical composition according to any one of claims 1 to 12 for use in the treatment of a disease selected from affective disorders, depression, major depressive disorder, postnatal depression, depression associated with bipolar disorder, Alzheimer's disease, psychosis, cancer, age or Parkinson's disease, anxiety, general anxiety disorder, social anxiety disorder, obsessive compulsive disorder, panic disorder, panic attacks, phobia, social phobia, agoraphobia, stress urinary incontinence, emesis, irritable bowel syndrome, eating disorders, chronic pain, partial responders, treatment resistant depression, Alzheimer's disease, cognitive impairment, attention deficit hyperactivity disorder, melancholia, posttraumatic stress disorder, hot flushes, sleep apnea, alcohol, nicotine or carbohydrate craving, substance abuse and alcohol and drug abuse.

15. A method for enhancing solubility of vortioxetine hydrobromide, **characterized in that** the vortioxetine hydrobromide is provided in a pharmaceutical composition as defined in any one of claims 1 to 12.
